# EUROPEAN PATENT APPLICATION

(11) **EP 4 230 193 A1**
(43) Date of publication of application: **23.08.2023**
(21) Application number: 22158046.7
(22) Date of filing: 22.02.2022
(51) Int. Cl.: A61K 9/00, A61K 9/08, A61K 31/382, A61K 31/498, A61K 47/32, A61K 47/38, A61P 27/02, A61P 27/06

(54) **OPHTHALMIC PHARMACEUTICAL COMPOSITION**

(71) Applicant: Warszawskie Zaklady Farmaceutyczne Polfa S.A., 01-207 Warszawa (PL)
(72) Inventor: Kacprzak, Katarzyna, 02-315 Warszawa (PL); Ratajczak, Tomasz, 02-315 Warszawa (PL); Czubowicz, Joanna, 02-315 Warszawa (PL); Borychowska, Joanna, 02-315 Warszawa (PL)
(74) Representative: Obrycki, Pawel Andrzej

(57) **Abstract**

The present invention relates to aqueous high viscosity topical ophthalmic compositions comprising brimonidine and dorzolamide and a polymer as a viscosity-enhancing agent.

## Description

### Field of the invention

The present invention relates to aqueous high viscosity topical ophthalmic compositions comprising brimonidine and dorzolamide and a polymer as a viscosity-enhancing agent.

### Background of the invention

Brimonidine is an alpha-adrenergic agonist and 2-imidazoline derivative. Brimonidine is used to treat open-angle glaucoma, ocular hypertension and rosacea. Brimonidine reduces an aqueous humor production and increases uveoscleral outflow. Because it is oxidatively stable, brimonidine is associated with fewer reports of ocular allergic reactions compared to other alpha-2 adrenergic agonists. Brimonidine is sold under the brand name Alphagan. the product contains benzalkonium chloride, poly(vinyl alcohol), sodium chloride, sodium citrate, citric acid monohydrate, purified water and hydrochloric acid or sodium hydroxide for pH adjustment.

In the US brimonidine is also available as over the counter (OTC) eye drops comprising brimonidine tartrate formulation in a concentration of 0.025%. It is intended to relieve redness in the sclera of the eyes for periods of up to eight hours at a time through its vasoconstrictive effects.

Dorzolamide is a non-bacteriostatic sulfonamide derivative and topical carbonic anhydrase (CA) inhibitor. It works by blocking an enzyme in the ciliary process that regulates ion balance and fluid pressure in the eyes. Dorzolamide is commercially available in Europe from Santen under the brand name Trusopt. Dorzolamide is indicated for the management of elevated intraocular pressure in patients with ocular hypertension or open-angle glaucoma. The marketed product composition contains hydroxyethyl cellulose, mannitol, sodium citrate, sodium hydroxide for pH adjustment and water for injections.

Dorzolamide can also be used in combination with timolol for the same indication in patients who are insufficiently responsive to ophthalmic beta-blockers.

Product containing dorzolamide and brimonidine is not approved on the European market. In the US, such product is manufactured and distributed by ImprimisRx. The product contains dorzolamide, brimonidine, sodium chloride, sodium citrate, Pluronic L64^{®} and sterile water.

Pluronic L64 is a non-ionic polyoxyethlene-polyoxypropylene block copolymer that is used widely in cosmetic compositions. The substance can be used as a solubilizing and suspending agent. However, Pluronic L64 is not available in Europe as a pharmaceutical-grade material. It can be used only for technical purposes.

Synperonic PE / L 64 (Poloxsamer 184) available on the European market as an equivalent of Pluronic 64 is in the form of a liquid material, difficult to prepare in the production process because it is highly heat sensitive. Due to its sensitivity to changes of temperature and pH, it is rheologically unstable, because even slight changes in temperature or shear force change its viscosity. The disadvantages of these polymers are weak mucoadhesive and poor mechanical properties, short residence time due to easily undergoing dissolution at the action site. Pluronic L64 will not be able to get a viscosity in the range of 75-150 cps.

Topical drug delivery is a preferred method for therapeutic treatment of most of ocular problems and a well-accepted route of administration for the treatment of various eye diseases. A major problem associated with topical drug delivery is poor ocular bioavailability due to anatomical and physiological constraints. This includes poor permeability of cornea, nasolacrimal drainage and short retention time in the precorneal area.

Topical delivery of drugs to the ocular tissue is affected by a complex of factors. Any change in formulation may have an impact on corneal penetration and ocular absorption. For example, viscosity of the pharmaceutical composition affects the mucosal retention of the active ingredient. Increased viscosity of the composition prolongs the retention time of the composition in the precorneal area. Normally, the drug contained in the drops is lost through the tear drainage. The drainage rates and the residence time of eye drops depend on the viscosity of the installed medication.

Therefore, an increase in residence time of a medication likely enhances therapeutic benefits. The viscosity of a pharmaceutical composition sometimes decreases with time due to decomposition of the polymer by light or heat. In such case the viscosity enhancing component can undergo conformational changes which will have an impact on its properties and in turn on bioavailability of the composition.

Ocular contact time, and hence the bioavailability of a drug can be enhanced by increasing the viscosity of the ophthalmic formulation.

Dorzolamide has limited aqueous solubility at physiologic pH, therefore the pH of Trusopt^{®} eye drops (containing 2% dorzolamide) has to be kept at about 5.65. Trusopt^{®} has a viscosity of 100 cps which increases the contact time of the drug with the eye surface thus increasing the topical bioavailability.

Sigurdsen et al. tested low viscosity formulations of dorzolamide. Dorzolamide hydrochloride was formulated as 2% and 4% low viscosity solutions (viscosity 3 to 5 cps, respectively) containing randomly methylated β-cyclodextrin at pH 7.45. These formulations were evaluated in rabbits. The dorzolamide concentrations in cornea, aqueous humor, iris-ciliary body, vitreous humour, retina and optic nerve were determined at various time points up to 8 hours of administration of the eye drops to rabbits. Trusopt^{®} having viscosity of 100 cps was used as a reference standard. The marketed formulation shows an initial rapid drop in intraocular pressure which lasts for three hours. The tested formulations showed lower concentrations of dorzolamide in aqueous humor compared to Trusopt^{®} within three hours upon administration. A 30- and 20- fold increase in the viscosity of the eye drop solution of Trusopt^{®} results in significant increase in the contact time of the drug with the eye surface and consequently enhanced topical availability. Improved dorzolamide bioavailability will improve the clinical efficacy of the drug *(*Maren TH (1995): The development of topical carbonic anhydrase inhibitors. J Glaucoma 4: 49- 62). Therefore, it is desirable to keep the viscosity of the dorzolamide solution in a certain range.

Thus, there is a need to develop a pharma grade stable composition comprising brimonidine and dorzolamide or pharmaceutically acceptable salts thereof having desirable viscosity that will not decrease in time and will lead to increased bioavailability of dorzolamide.

### Summary of the invention

The present invention is directed toward aqueous ophthalmic compositions for topical administration that comprise dorzolamide, brimonidine and a viscosity enhancing component selected from hydroxyethyl cellulose, sodium carboxymethyl cellulose, polyvinylpyrrolidone and mixtures thereof.

Surprisingly, it was found that compositions comprising brimonidine and dorzolamide together with a viscosity enhancing component selected from hydroxyethyl cellulose, sodium carboxymethyl cellulose polyvinylpyrrolidone and mixtures thereof are high viscous, stable and provide optimal ocular absorption.

### Description of the invention

Unless otherwise indicated, all ingredient concentrations are listed as a weight/volume percentage basic (%w/v).

The ophthalmic compositions of the present invention are aqueous compositions. The compositions of the present invention are preferably not formulated as solutions that gel upon administration to the eye.

Accordingly, the present invention provides an aqueous topical ophthalmic pharmaceutical composition comprising brimonidine or pharmaceutically acceptable salt thereof and dorzolamide or pharmaceutically acceptable salt thereof and a viscosity enhancing agent that is selected from the group consisting of hydroxyethyl cellulose, sodium carboxymethyl cellulose, polyvinylpyrrolidone and mixtures thereof.

Dorzolamide is preferably used in composition of the present invention in the form of a salt. Examples of pharmaceutically acceptable salts of dorzolamide include inorganic salts such as hydrochloride, hydrobromide, sulphate. Preferably, dorzolamide is used in the present invention in the form of hydrochloride salt.

Dorzolamide or pharmaceutically acceptable salt thereof is preferably present in the composition according to the present invention in an amount of 1.5 to 2%, wherein the used amount corresponds to dorzolamide free base. In a preferred embodiment of the present invention, dorzolamide or a pharmaceutically acceptable salt thereof is present in the composition in an amount of 2 % (w/v), wherein the used amount corresponds to dorzolamide free base.

Brimonidine is preferably used in the composition of the present invention in the form of a salt. Preferably, brimonidine is in the form of tartrate salt.

Brimonidine or pharmaceutically acceptable salt thereof is preferably present in the composition according to the present invention in an amount of 0.025 to 0.2 % (w/v). In a preferred embodiment of the present invention, brimonidine or pharmaceutically acceptable salt thereof is present in the composition in an amount of 0.2 % (w/v).

Hydroxyethyl cellulose (HEC) is a nonionic water-soluble polymer widely used in pharmaceutical formulations and cosmetics. It is available in several grades that vary in viscosity and degree of substitutions. HEC is commercially available from the Ashland Inc. Company under the brand name Natrosol e.g. 250 MR, 250 H4BR, 250 HHBR, MHBR, MBR, 250 HX-Pharm. Most preferred for use in the compositions of the present invention is Natrosol 250 HX-Pharm which has an average molecular weight of approximately 1,000,000 Daltons. The concentration of HEC in the compositions of the present invention will range from 0.05 to 0.5 %, and will preferably be 0.5 %.

Carboxymethyl cellulose sodium (CMC - Na) is an anionic polymer widely used in oral and topical formulations, primarily for its viscosity increasing properties. Typical molecular weight of sodium carboxymethylcellulose is 90,000-700,000. Most preferred for use in the composition of the present invention is CEKOL 700, which has an average molecular weight of approximately 270,000 Daltons and is available from CP Kelco Company. The concentration of CMC-Na in the composition of the present invention will range from 0.5 to 1.5 % , and will preferably be 1.3%.

Polyvinylpyrrolidone (PVP) is also known as K90. It is a water-soluble polymer obtained by polymerization of monomer *N*-vinylpyrrolidone. It is available from BASF Company. It is chemically stable and has low toxicity. The concentration of PVP in the composition of the present invention will range from 0.4 to 0.7 % , and will preferably be 0.6 %.

When used, excipients and carriers incorporated into the composition of the invention must be ophthalmologically acceptable. "Ophthalmologically acceptable excipients or carriers" refers to ophthalmologically acceptable materials, compositions or vehicles. Each component must be ophthalmologically acceptable in the sense of being compatible with the other ingredients of the ophthalmic composition.

The pharmaceutical composition of the present invention preferably comprises at least one further excipient selected from the group consisting of buffering agents, pH adjusting agents and tonicity agents. In the preferred embodiment of the present invention the composition comprises buffering agents, tonicity agents and pH adjusting agents.

Ophthalmic composition of the present invention may include an effective amount of a buffering agent. Examples of buffering agents that may be used in the ophthalmic composition of the present invention include citric acid, citrates, phosphoric acid, phosphates, acetates and mixtures thereof. In a preferred embodiment of the present invention the buffering agent is selected form citric acid, sodium citrate, phosphoric acid, sodium or potassium phosphate, dibasic sodium phosphate, sodium or potassium acetate and mixtures thereof. The most preferred buffering agent used in the ophthalmic composition of the present invention is sodium citrate. The buffer provides pH of the eye drop solution in the range of 6.0-7.0. The pH range of the product is in accordance with U.S. Pharmacopeia (current edition) chapter Ophthalmic Products <771>, which is 3.0÷8.6, depending on formulation. The pH of tear fluid is about 7.4; the eye can tolerate pH of a product in the range mentioned.

Ophthalmic composition of the present invention may include an effective amount of tonicity agents. Examples of tonicity agents that may be used in the ophthalmic composition of the present invention include sodium chloride, mannitol, glycerin, sorbitol and mixtures thereof. In a preferred embodiment of the present invention the tonicity agent is mannitol. The tonicity agent of the present invention may be used in the concentration ranging from 0.1 to 4.7 % (w/v), preferably 0.1 to 2.3 % (w/v), most preferably 2.3. % (w/v).

Compositions of the present invention may contain a pH adjusting agent, preferably in an amount sufficient to obtain a composition having a pH of 5-6. Preferably, compositions of the present invention have a pH of 5.4- 6.5., more preferably 6.4. Strong acids such as hydrochloride acid and strong bases such as sodium hydroxide may be used to adjust pH.

In one embodiment the ophthalmic composition of the present invention consists of dorzolamide hydrochloride, brimonidine tartrate, hydroxyethyl cellulose, mannitol, sodium citrate, sodium hydroxide, hydrochloric acid and purified water.

In another embodiment the ophthalmic composition of the present invention consists of dorzolamide hydrochloride, brimonidine tartrate, sodium carboxymethyl cellulose, mannitol, sodium citrate, sodium hydroxide, hydrochloric acid and purified water.

In another embodiment the ophthalmic composition of the present invention consists of dorzolamide hydrochloride, brimonidine tartrate, polyvinylpyrrolidone, mannitol, sodium citrate, sodium hydroxide, hydrochloric acid and purified water.

According to a particularly preferred aspect, the composition according to the invention is devoid of preservatives. By preservative it is understood any substance which can be used as a ophthalmological preservative, e.g. benzalkonium chloride, polyquaternium-1, thimerosal, phenyl mercuric nitrate, phenyl mercuric acetate, chlorbutanol, benzyl alcohol, sorbic acid, methyl/propyl paraben, disodium EDTA. It has been recognized that such preservatives may cause many side effects including irritation of the eye caused by damage of the epithelial cells.

The ophthalmic composition of the present invention can be packed in suitable single dose containers or a multi-dose containers. In a preferred embodiment of the present invention the composition is packed in a low density polyethylene container (LDPE), advantageously of EP quality containing no additives. Non-limiting examples of multi-dose container to deliver eye drops without preservatives for several days are Novelia^{®} system or 3K^{®} system.

The viscosity of the pharmaceutical composition of the present invention is preferably 20 to 150 mPa s, more preferably 75 to 120 mPa ·s. In a preferred embodiment, the composition of the present invention has a viscosity of 80 to 115 mPa ·s. The viscosity is a value measured by methods known to those skilled in the art, for example by a rotational viscometer (Brookfield type DV3T) equipped with SC4-31 or SC4-18 spindles compliant with the requirements of European Pharmacopeia 2.2.10.

Furthermore, the present invention relates to a process for the preparation of the ophthalmic composition as described above. The ophthalmic composition of the present invention may be prepared by the process which comprises the following steps:
a) filling a container with purified water at the temp. 25°C;
b) dissolving hydroxyethyl cellulose and/or sodium carboxymethyl cellulose and/or polyvinylpyrrolidone in water and mixing;
c) sterilization at 121°C for 30 minutes;
d) filling another container with purified water at the temp. 25°C and adding dorzolamide hydrochloride, tonicity agent and buffer and mixing;
e) adding brimonidine tartrate to the solution obtained in step d);
f) optionally adjusting of pH of the solution as obtained in step (e) with a pH adjusting agent;
g) optionally filtering of the solution as obtained in step (f), preferably through 0.2 µm membrane directly to the container with solution comprising hydroxyethyl cellulose and/or sodium carboxymethyl cellulose and/or polyvinylpyrrolidone;
h) adjusting the volume of the obtained solution to 1 ml with purified water;
i) optionally transferring the obtained solution to sterile containers.

The following examples are provided to illustrate different aspects and embodiments of the present invention. These examples are not intended to limit in any way the disclosed invention. The following abbreviations and definitions have been used in the examples:
**Carbopol 974P** belongs to the family of Carbomers which are acrylic acid polymers that are used for a variety of purposes. They are used in pharmaceutical processes as suspending agents, gel bases, emulsifiers, and binding agents. They are also used as artificial tears. Carbomer 974P is a carbomer that is the major non-aqueous component (5% polymer, 94% water) of a proprietary formulation called BufferGel, which also contains dibasic potassium phosphate, magnesium sulfate, dibasic sodium phosphate, sorbic acid, monobasic sodium phosphate, and disodium EDTA. Carbopol 974P is commercially available from Lubrizol.
**Pluronic L64^{®} (Poloxamer)** is a difunctional block copolymer surfactant terminating in primary hydroxyl groups. The substance can be used as a solubilizing and suspending agent. Synperonic PE / L 64 (Poloxsamer 184) is an equivalent of Pluronic 64 commercially available from CRODA.

### Example 1 - composition A

The ophthalmic composition of the present invention may be prepared by the process which comprises the following steps:
a) A container was filled with purified water at the temp. 25°C, then 5.26 mg of hydroxyethyl cellulose was dissolved while continuously mixing. Dissolution was continued for 2 hours. The solution was then sterilized at 121°C for 30 minutes;
b) Another container was filled with highly purified water at the temp. 25°C, then 23 mg of mannitol, 2.94 mg of sodium citrate and 22.26 mg of dorzolamide hydrochloride were dissolved while continuously mixing. Dissolution was continued for 30 minutes;
c) 2 mg of brimonidine tartrate was added to the solution obtained in step b). Dissolution was continued for 40 minutes;
d) pH of the solution obtained in step (c) was adjusted to 6.4 by adding sodium hydroxide/ hydrochloric acid (1 N);
e) the solution obtained in step (d) was filtered through 0.2 µm membrane to the container with solution obtained in step a);
f) the volume of the solution obtained in step e) was adjusted to 1 ml with purified water;
g) the solution obtained in step f) was transferred to sterile containers.

**Table 1. Composition A**

| Ingredients | mg/ml |
|---|---|
| dorzolamide hydrochloride | 22.26* |
| brimonidine tartrate | 2 |
| mannitol | 23 |
| hydroxyethyl cellulose | 5.25 |
| sodium citrate | 2.94 |
| sodium hydroxide | adjust pH |
| hydrochloric acid | adjust pH |
| purified water | at 1 ml |

| | |
|---|---|
| *22.26 mg/ml dorzolamide hydrochloride is equivalent to 20 mg/ml of dorzolamide free base | |

### Example 2 - composition B

a) A container was filled with purified water at the temp. 25°C, then 13 mg of sodium carboxymethyl cellulose was dissolved while continuously mixing. Dissolution was continued for 1 hours. The solution was then sterilized at 121°C for 30 minutes;
b) Another container was filled with highly purified water at the temp. 25°C, then 23 mg of mannitol, 2.94 mg of sodium citrate and 22.26 mg of dorzolamide hydrochloride were dissolved while continuously mixing. Dissolution was continued for 30 minutes;
c) 2 mg of brimonidine tartrate was added to the solution obtained in step b). Dissolution was continued for 40 minutes;
d) pH of the solution obtained in step (c) was adjusted to 6.4 by adding sodium hydroxide/ hydrochloric acid (1 N);
e) The solution obtained in step (d) was filtered through 0.2 µm membrane to the container with solution obtained in step a);
f) the volume of the solution obtained in step e) was adjusted to 1 ml with purified water;
g) the obtained solution was transferred to sterile containers.

**Table 2. Composition B**

| Ingredients | mg/ml |
|---|---|
| dorzolamide hydrochloride | 22.26* |
| brimonidine tartrate | 2 |
| mannitol | 23 |
| sodium carboxymethyl cellulose | 13 |
| sodium citrate | 2.94 |
| sodium hydroxide | adjust pH |
| hydrochloric acid | adjust pH |
| purified water | at 1 ml |

| | |
|---|---|
| *22.26 mg/ml dorzolamide hydrochloride is equivalent to 20 mg/ml of dorzolamide free base | |

### Example 3 - Comparative example- composition C

The composition was prepared in the following process:
a) A container was filled with purified water at the temp. 25°C, then 1.4 mg of Carbopol 974P was dissolved while continuously mixing. Dissolution was continued for 1 hours. The solution was then sterilized at 121°C for 15 minutes;
b) Another container was filled with purified water at the temp. 25°C, then 23 mg of mannitol, 2.94 mg of sodium citrate and 22.26 mg of dorzolamide hydrochloride were dissolved while continuously mixing. Dissolution was continued for 30 minutes;
c) 2 mg of brimonidine tartrate was added to the solution obtained in step b). Dissolution was continued for 40 minutes;
d) pH of the solution obtained in step (c) was adjusted to 6.4 by adding sodium hydroxide/ hydrochloric acid (1 N);
e) The solution obtained in step (d) was filtered through 0.2 µm membrane to the container with solution obtained in step a);
f) The volume of the solution obtained in step e) was adjusted to 1 ml with purified water;
g) the obtained solution was transferred to sterile containers.

**Table 3. Composition C**

| Ingredient | mg/ml |
|---|---|
| dorzolamide hydrochloride | 22.26* |
| brimonidine tartrate | 2 |
| mannitol | 23 |
| Carbopol 974P | 1.4 |
| sodium citrate | 2.94 |
| sodium hydroxide | adjust pH |
| hydrochloric acid | adjust pH |
| purified water | at 1 ml |

| | |
|---|---|
| *22.26 mg/ml dorzolamide hydrochloride is equivalent to 20 mg/ml of dorzolamide free base | |

### Example 4

a) The container was filled with purified water at the temp. 20-25°C and heated to 30°C, then 2.94 mg of sodium citrate dihydrate and 7 mg sodium chloride were dissolved while continuously mixing;
b) 2.25 mg of brimonidine tartrate was added to the solution obtained in step a) and mixed;
c) pH of the solution obtained in step b) was adjusted to 7 by sodium hydroxide;
d) 2.26 mg of dorzolamide hydrochloride was added to the solution obtained in step c) and mixed;
e) 100 mg of Pluronic L64^{®} was added to the solution obtained in step d) ;
f) pH of the solution obtained in step e) was adjusted to 5.7;
g) purified water was added to the solution obtained in step f) to bring the final volume to 1 ml;
h) the obtained solution was filtered through 0.2 µm membrane.

**Table 4. Comparative example - Composition D**

| Ingredient | mg/ml |
|---|---|
| dorzolamide hydrochloride | 22.26* |
| brimonidine tartrate | 2.25 |
| sodium chloride | 5.54 |
| Pluronic L64 | 100 |
| sodium citrate dihydrate | 1.5 |
| sodium hydroxide | Adjust pH |
| hydrochloric acid | Adjust pH |
| purified water | at 1 ml |

| | |
|---|---|
| *22.26 mg/ml dorzolamide hydrochloride is equivalent to 20 mg/ml of dorzolamide free base | |

### Example 5 - Comparative example - composition E

A composition was prepared using the following process:
a) The container was filled with purified water at the temp. 20-25°C and heated to 30°C, then 2.94 mg of sodium citrate dihydrate and 7 mg sodium chloride were dissolved while continuously mixing;
b) 2.25 mg of brimonidine tartrate was added to the solution obtained in step a) and mixed;
c) pH of the solution obtained in step b) was adjusted to 7 by sodium hydroxide;
d) 2.26 mg of dorzolamide hydrochloride was added to the solution obtained in step c) and mixed;
e) 250 mg of Pluronic L64^{®} was added to the solution obtained in step d);
f) pH of the solution obtained in step e) was adjusted to 5.7;
g) purified water was added to bring to the solution obtained in step f) to bring the final volume to 1 ml;
h) the obtained solution was filtered through 0.2 µm membrane.

**Table 5. Comparative example - Composition E**

| Ingredient | mg/ml |
|---|---|
| dorzolamide hydrochloride | 22.26* |
| brimonidine tartrate | 2.25 |
| sodium chloride | 5.54 |
| Pluronic L64 | 250 |
| sodium citrate dihydrate | 1.5 |
| sodium hydroxide | Adjust pH |
| hydrochloric acid | Adjust pH |
| purified water | at 1 ml |

| | |
|---|---|
| *22.26 mg/ml dorzolamide hydrochloride is equivalent to 20 mg/ml of dorzolamide free base | |

### Example 6 - Composition F

a) A container was filled with purified water at the temp. 25°C, then 60 mg of polyvinylpyrrolidone was dissolved while continuously mixing. Dissolution was continued for 1 hours. The solution was then sterilized at 121°C for 15 minutes;
b) another container was filled with purified water at the temp. 25°C, then 20 mg of mannitol, 2.94 mg of sodium citrate and 22.26 mg of dorzolamide hydrochloride were dissolved while continuously mixing. Dissolution was continued for 30 minutes;
c) 2 mg of brimonidine tartrate was added to the solution obtained in step b). Dissolution was continued for 40 minutes;
d) pH of the solution obtained in step (c) was adjusted to 6.4 by adding sodium hydroxide/ hydrochloric acid (1 N);
e) the solution obtained in step (d) was filtered through 0.2 µm membrane to the container with the solution obtained in step a);
f) the volume of the solution obtained in step e) was adjusted to 1 ml with purified water;
g) the obtained solution was transferred to sterile containers.

**Table 6. Example 6 - Composition F**

| Ingredients | mg/ml |
|---|---|
| dorzolamide hydrochloride | 22.26* |
| brimonidine tartrate | 2 |
| mannitol | 20 |
| polyvinylpyrrolidone | 60 |
| sodium citrate | 2.94 |
| sodium hydroxide | Adjust pH |
| hydrochloric acid | Adjust pH |
| purified water | at 1 ml |

| | |
|---|---|
| *22.26 mg/ml dorzolamide hydrochloride is equivalent to 20 mg/ml of dorzolamide free base | |

### Results

### Degree of coloration of liquids.

Incompatibility studies were performed in order to determine interactions of the drug substance with viscosity increasing components planned to be used in the final formulation. Composition shown in Tables 1 -6 were prepared and degree of coloration of liquids was examined by visual method according to the standard of PH. Eur. 2.2.1. The colors were compared in diffused daylight viewing horizontally (method I) and vertically (method II) against a white background.

**Table 7. Results of incompatibility studies**

| **Composition** | **Degree of coloration of liquids** |
|---|---|
| Composition A | transparent |
| Composition B | transparent |
| Composition C | opaque suspension |
| Composition D | transparent |
| Composition E | transparent |
| Composition F | transparent |

Substantial changes in physical appearance of the product were observed in composition C. While composition A, B, D, E and F were transparent, composition C was a cloudy suspension and precipitation was observed. This suggest incompatibility of a polymer used in the composition with at least one active substance. Such composition may lead to undesired change in the performance of the drug. This data suggests that Carbopol 974P cannot be used as a viscosity enhancing excipient.

### Viscosity data

Composition C proved incompatible with active ingredients, therefore only compositions A, B, D, E and F were taken for further analysis. The composition shown in Table 8 were prepared and their viscosity was measured at 20°C using a rotational viscometer (Brookfield type DV3T) equipped with SC4-31 or SC4-18 spindles according to in-house method compliant with the requirements of European Pharmacopeia 2.2.10.

**Table 8. Viscosity measured at 20°C**

| **Composition** | **Viscosity (mPa·s)** |
|---|---|
| Composition A | 103.5 |
| Composition B | 100.2 |
| Composition D | 2.84 |
| Composition E | 13.7 |
| Composition F | 128.1 |

These data suggest that compositions with Pluronic L64 (composition D and E respectively) do not have viscosity that provides sufficient therapeutic effect of dorzolamide as according to the literature data the viscosity of a composition containing dorzolamide need to be between 75 and 150 mPa s. Consequently, the time of retention of such composition on the eye surface is decreased and the drug contained in the drops is lost due to absorption through the conjunctiva or through the tear drainage.

Since compositions D and E do not have required viscosity only compositions A, B, F were taken for further analysis.

Stability studies were performed using tested solutions i.e. composition of Example 1, composition of Example 2 and composition of Example 6. The tested solutions were filled in a multi-dose containers and kept for three months under the following conditions:
- at 25°C and a relative humidity of 60%,
- at 40°C and a relative humidity of 75%.

Viscosity of the tested solutions was measured at 20°C using a rotational viscometer (Brookfield type DV3T) equipped with SC4-18 spindles. The viscosity was measured after one month and 3 months of storage.

**Table 8. Viscosity measured after 1 and 3 months of storage.**

| **Time and conditions** | **Composition A** | **Composition B** | **Composition F** |
|---|---|---|---|
| | **Viscosity (mPa · s)** | **Viscosity (mPa ·s)** | **Viscosity (mPa ·s)** |
| **1 month** | 98.7 | 100.2 | 127.5 |
| **25°C and a relative humidity of 60%** | | | |
| **3 month** | 97.3 | 83.5 | 127.3 |
| **25°C and a relative humidity of 60%** | | | |
| **1 months** | 85.5 | 93.6 | 125.2 |
| **40°C and a relative humidity of 75%.** | | | |
| **3 months** | 82.5 | 86.6 | 125.0 |
| **40°C and a relative humidity of 75%.** | | | |

The content of both active ingredients during 3 months of storage did not decrease for all three compositions and was in the range of 99% for dorzolamide and 98% for brimonidine.

These results suggest that both compositions are stable and have viscosity which provides optimal absorption/bioavailability of dorzolamide. Both viscosity enhancing components are commercially available as a pharma-grade material.

Documents cited in the application.
Sigurdsen et al. Cyclodextrin formulation of dorzolamide and its distribution in the eye after topical administration. Journal of Controlled Release, 2005, 102: 255-262.
Maren et al. The development of topical carbonic anhydrase inhibitors. J Glaucoma 1995, 4: 49- 62.
European Pharmacopoeia 10.0 Volume I published July 2019
U.S. Pharmacopeia

## Claims

1. An aqueous topical ophthalmic composition comprising dorzolamide or pharmaceutically acceptable salts thereof and brimonidine or pharmaceutically acceptable salts thereof and a polymer as a viscosity enhancing component selected from the group consisting of hydroxyethyl cellulose, sodium carboxymethyl cellulose, polyvinylpyrrolidone and mixtures thereof.

2. The composition according to claim 1, wherein the dorzolamide is dorzolamide hydrochloride.

3. The composition according to claim 1, wherein the brimonidine is brimonidine tartrate.

4. The composition according to claims 1 or 2, wherein dorzolamide or pharmaceutically acceptable salts thereof is present in the composition in an amount of 1.5 to 2 % (w/v), preferably 2 % (w/v), wherein the used amount corresponds to dorzolamide free base.

5. The composition according to claims 1 or 3, wherein brimonidine or pharmaceutically acceptable salts thereof is present in the composition in an amount 0.025 to 0.2% (w/v), preferably 0.2 % (w/v).

6. The composition according to any of the preceding claims, wherein the composition further comprises one or more ingredients selected form the group consisting of buffering agents, tonicity agents and pH adjusting agents.

7. The composition according to claim 6, wherein the composition comprises a buffering agent selected from the group consisting of citric acid, phosphoric acid, phosphates, citrates, acetates and mixtures thereof, preferably citric acid, phosphoric acid, sodium or potassium phosphate, dibasic sodium phosphate, sodium or potassium acetate and mixtures thereof, more preferably sodium citrate.

8. The composition according to claim 6, wherein the composition comprises a tonicity agent selected from the group consisting of sodium chloride, mannitol, glycerin, sorbitol and mixtures thereof, preferably mannitol.

9. The composition according to claim 6, wherein the composition has a pH of 5 to 6, preferably 5.4.

10. The composition according to any of the proceeding claims, wherein the composition consists of:
- brimonidine tartrate
- dorzolamide hydrochloride
- hydroxyethyl cellulose
- mannitol
- sodium citrate
- sodium hydroxide
- hydrochloric acid
- purified water

11. The composition according to claims 1-9, wherein the composition consists of:
- brimonidine tartrate
- dorzolamide hydrochloride
- sodium carboxymethyl cellulose
- mannitol
- sodium citrate
- sodium hydroxide
- hydrochloric acid
- purified water

12. The composition according to claims 1-9, wherein the composition consists of:
- brimonidine tartrate
- dorzolamide hydrochloride
- polyvinylpyrrolidone
- mannitol
- sodium citrate
- sodium hydroxide
- hydrochloric acid
- purified water

13. The composition according to claims 1-10, wherein the hydroxyethyl cellulose is present in an amount of 0.05 to 0.5 % (w/v), preferably 0.5 % (w/v)

14. The composition according to claim 1-9 and 11, wherein the sodium carboxymethyl cellulose is in an amount of 0.5 to 1.5 % (w/v) , preferably 1.3% (w/v).

15. The composition according to claim 1-9 and 12 wherein the polyvinylpyrrolidone is in an amount of 0.4 to 0.7 (w/v).
